# EUROPEAN PATENT SPECIFICATION

(11) **EP 3 074 065 B1**
(45) Date of publication and mention of the grant of the patent: **26.07.2023**
(21) Application number: 13898176.6
(22) Date of filing: 27.11.2013
(51) Int. Cl.: A61M 5/32

(54) **FRONTAL ATTACHMENT DEVICE FOR SYRINGE WITH ROTATIONALLY ACTIVATED RETRACTABLE NEEDLE**
STIRNSEITIGE BEFESTIGUNGSVORRICHTUNG FÜR EINE SPRITZE MIT EINER DURCH DREHUNG AKTIVIERTEN EINZIEHBAREN NADEL
DISPOSITIF DE FIXATION FRONTALE POUR SERINGUE AVEC AIGUILLE RÉTRACTABLE ACTIVÉE PAR ROTATION

(30) Priority: 26.11.2013 US 201314091113
(43) Date of publication of application: 05.10.2016
(73) Proprietor: Retractable Technologies, Inc., Little Elm, TX 75068 (US); Shaw, Thomas J., Frisco, TX 75034 (US)
(72) Inventor: SHAW, Thomas J., Frisco, TX 75034 (US); SMALL, Mark, Heaverner, OK 74937 (US); ZHU, Ni, McKinney, TX 75071 (US)
(74) Representative: Grünecker Patent- und Rechtsanwälte PartG mbB
(86) International application number: PCT/US2013/072218
(87) International publication number: WO 2015/080724

(56) References cited:
- WO-A1-2014/093026
- WO-A2-03/099349
- JP-A- H10 179 738
- US-A- 5 445 618
- US-A- 5 445 618
- US-A1- 2002 082 560
- US-A1- 2002 082 560
- US-A1- 2009 306 601
- US-A1- 2009 306 601
- US-A1- 2012 078 225
- US-A1- 2012 078 225

## Description

### CROSS-REFERENCE TO RELATED APPLICATIONS

### BACKGROUND OF THE INVENTION

### 1 . Field of the Invention

This invention relates to the use of conventional syringes in combination with a newly disclosed frontal attachment having a retractable needle. The invention relates more particularly to a medical device comprising in combination a needle, nose and hub assembly attachable to a conventional syringe preferably having a forwardly facing luer lock connector. The subject device has a needle retraction mechanism that is activated by rotating the syringe barrel relative to the nose of the device to retract the needle from a patient and propel it into the nose and a retraction tube that is part of the hub assembly.

### 2. Description of Related Art

Conventional syringes comprising a generally cylindrical barrel, a fixed needle projecting forwardly from the barrel, and a plunger slidably disposed inside the barrel through an opening in the rear of the barrel are well known. More recently, syringes have been made with a luer connector on the front of the barrel to which a needle hub is attachable to allow needles of different gauges or sizes to be used with a commonly configured barrel.

Even more recently, in an effort to control the spread of blood-borne pathogens and the incidence of contamination by contact with either exposed needles or bodily fluids, syringes having fixed or changeable needles have been designed to embody various "safety" elements. Such "safety" elements should desirably include a retractable needle, but many products marketed as having "safety" elements include, for example, covers or guards that are manually operated by medical personnel administering an injection to shield or cover the needle tip following removal of the needle from a patient.

Some previously disclosed needle retraction systems without changeable needles are activated either manually or automatically by application of a force upon completion of an injection to force the needle and needle tip back inside a retraction chamber. The only known syringe having a changeable, retractable needle does not have a conventional luer lock connection, and the retraction mechanism is activated by the application of a forwardly directed force to the plunger handle following removal of the needle from a patient, thereby exposing the needle and also exposing the user to the risk of needle-stick injury.

U.S. Pub. No. 2006/0155244 to Popov discloses a venipuncture device that rotates a port unit following needle retraction. The retraction chamber is disposed inside the medical device, is not part of the frontal attachment and remains stable while the port unit is moved rotationally relative to the retraction chamber following needle retraction. The frontal attachment disclosed there cannot be used with a generic luer lock syringe.

Although many advancements in syringe technology have been made in recent years, a frontal attachment device is needed that can be used with a standard syringe having a conventional luer lock connector, that offers the advantages of a changeable needle in combination with the advantages of a retractable needle providing sufficient retraction force to retract the needle while inserted into a patient, and that can be activated by the application of a rotational force to the syringe barrel while stabilizing the nose without applying a forwardly directed force to the plunger or the needle.

In US 2009/306601 A1 there are two different configurations of a retractable needle assembly known. In one configuration, a part in housing is provided, which may be moved from a position, wherein the fluid can be extracted by the needle to the position, where the needle is retracted. In both positions, the needle is coaxially aligned with the retraction mechanism or the respective hub and the barrel. In a second embodiment, an arc-formed movement from a first position to a second position is disclosed.

Further, from WO 2014/093026 A1, a retractable needle assembly is known. In this needle assembly, the needle may move from a first position, wherein the needle protrudes out of the device to a second position via a lateral movement along a straight line. From JP H10 179738 A, a needle is known, which is retracted into a cavity of the barrel. Further configurations concerning medical devices are disclosed in WO 03/099349 A2, US 2002/082560 A1, US 5 445 618 A and US 2012/078225 A1.

### SUMMARY OF THE INVENTION

The invention is defined in claim 1. Advantageous embodiments are subject of the dependent claims.

A medical device is disclosed that comprises a hub assembly selectively attachable to the front of a conventional syringe, a nose projecting forwardly from the hub assembly, a rearwardly biased needle retraction mechanism seated inside the nose, a retractable needle projecting forwardly of the nose, and a fluid flow path from the fluid chamber through the hub assembly, nose, needle retraction mechanism and needle, wherein the needle is retracted following use into the nose and a retraction tube external to the syringe by rotating the syringe relative to the nose. As used throughout this disclosure, the terms "attachable," "detachable" and "changeable" are generally used to characterize frontal attachments, such as needles or needle/ hub combinations, that are selectively attachable to, detachable from, or otherwise changeable in relation to a syringe for purposes such as, without limitation, selecting a particular gauge needle for a particular clinical use.

A medical device as disclosed here can be configured to be attachable to the front of a conventional syringe having a luer lock connector. If desired, a medical device as disclosed here can also be made with a snap-on or other attachment mechanism instead of a conventional luer lock connector provided that the syringe with which it is used is cooperatively configured. A medical device as disclosed here desirably comprises a needle that is retractable, and retraction is activated by applying rotational rather than axial force to the syringe barrel. A satisfactory medical device as disclosed here desirably has sufficient retraction force to retract a needle that is still inserted into a patient's body and thereby prevents exposure of the contaminated needle to others. A medical device as disclosed here desirably utilizes a retraction tube that is not embodied in the syringe or plunger handle, and is instead part of the attachable hub assembly.

A satisfactory hub assembly for use in the invention as disclosed here comprises a hub disposed forwardly of the plunger, a retraction tube laterally spaced apart from the hub, and a frame member interconnecting the hub and the retraction tube in substantially fixed, laterally spaced-apart relation to each other. A medical device made in accordance with the invention desirably further comprises a nose projecting forwardly from the hub and is attached to the frame so as to permit the hub assembly to move laterally in relation to the nose between a first position axially aligned with the hub and a second position axially aligned with the retraction tube. A rearwardly biased needle retraction mechanism comprising a needle holder and retraction spring is desirably seated inside the nose; and a retractable needle projects forwardly of the nose. A fluid flow path is thereby provided from the fluid chamber of a syringe through the hub, nose, needle retraction assembly and needle; and an annular fluid seal is desirably disposed around a portion of the fluid flow path between the hub and the nose.

Following an injection using a syringe provided with the medical device disclosed here, a clinician administering the injection desirably grasps a stabilizer tab provided at the nose of the device with the thumb and finger or fingers of one hand to stabilize the body and nose, and with the other hand, rotates the syringe barrel in a clockwise direction (the same direction in which the barrel is rotated during attachment of the syringe to the device prior to use). As the body and nose are stabilized, the syringe barrel and the attached hub assembly are rotated relative to the nose, which first causes the hub to slide laterally out of coaxial alignment with the head of the needle holder. As the hub slides out of engagement with the rearwardly biased needle holder, preferably in a curvilinear arc, a portion of the frame member disposed between and connecting the hub to the laterally spaced-apart retraction tube comes into abutting engagement with the needle holder to continue holding the retraction spring in its compressed position. The needle holder remains rearwardly biased until such time as a forwardly facing opening into the retraction tube sufficiently approaches coaxial alignment with the needle holder to allow the compressed retraction spring to propel the needle holder rearwardly into the retraction tube. As this occurs, the needle holder also carries the attached needle rearwardly to a fully retracted position where the needle is released from the patient and the needle tip no longer projects forwardly from the nose.

In this way, the medical device disclosed here is configured to convert rotational motion of the syringe to curvilinear translational realignment of the nose and the needle holder from coaxial alignment with the hub to coaxial alignment with the retraction tube.

### BRIEF DESCRIPTION OF THE DRAWINGS

The apparatus of the invention is further described and explained in relation to the following drawings wherein:
FIG. 1 is a side elevation view of one embodiment of a syringe having selectively attached to its front end one embodiment of a medical device comprising in combination a nose, a needle or cannula projecting forwardly of the nose, a needle retraction mechanism seated inside the nose, and a hub assembly;
FIG. 2 is a cross-sectional view of the apparatus of FIG. 1 taken along line 2-2 of FIG. 9;
FIG. 3 is an exploded perspective view of the apparatus of FIG. 1;
FIG. 4 is an enlarged detail view of the attachable medical device of FIG. 1;
FIG. 5 is a side elevation view of the apparatus of FIG. 1 with the syringe plunger advanced to the post-injection position and the hub assembly rotated clockwise relative to the nose;
FIG. 6 is a cross-sectional view of the apparatus of FIG. 5 taken along line 6-6 of FIG. 10;
FIG. 7 is a side elevation view of the apparatus of FIG. 5 with the plunger advanced to the post-injection position and the hub assembly rotated further clockwise relative to the nose;
FIG. 8 is a cross-sectional view of the apparatus of FIG. 5 taken along line 6-6 of FIG. 10;
FIG. 9 is a front elevation view of the apparatus of FIG. 1;
FIG. 10 is a front elevation view of the apparatus of FIG. 5;
FIG. 11 is a front elevation view of the apparatus of FIG. 7;
FIG. 12 is a rear elevation view of the apparatus of FIG. 1;
FIG. 13 is a rear elevation view of the apparatus of FIG. 5;
FIG. 14 is a rear elevation view of the apparatus of FIG. 7; and
FIGS. 15-17 are right, left side and front elevation views, respectively, of the medical device of FIG. 1, with a needle cover installed prior to use.

Like reference numerals are used to describe like features in all Figures of the drawings.

### DESCRIPTION OF THE PREFERRED EMBODIMENTS

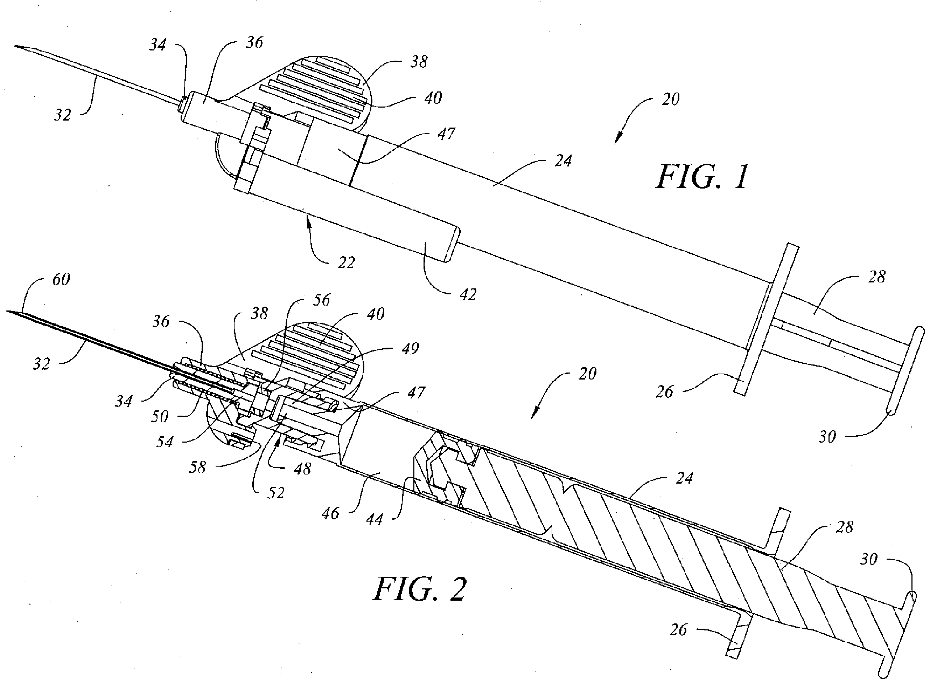

FIGS. 1 and 2 depict an assembled combination 20 of syringe barrel 24, having a luer lock connector 47, 49 disposed at the front end and a plunger handle 28 with plunger seal 44 slidably engaging the inside wall of syringe barrel 24, and a satisfactory medical device 22 of the invention having a hub assembly 48 that is attached to luer lock connector 47, 49 so as to establish fluid communication between fluid chamber 46 of syringe barrel 24 and needle 32. Plunger handle 28 extends rearwardly from an opening in the back of syringe barrel 24 and is positioned relative to syringe barrel 24 as it could be prior to the injection of a fluid disposed inside fluid chamber 46 into a patient if tip 60 of needle 32 were inserted into the patient, recognizing that the position of plunger seal 44 relative to barrel 24 will depend upon the amount of fluid that is drawn into fluid chamber 46. The injection force is applied to a fluid disposed inside fluid chamber 46 through plunger handle 28 by depressing thumb cap 30 of plunger handle 28 while stabilizing syringe barrel 24 by applying oppositely directed finger force to the forwardly facing surfaces of outwardly projecting flanges 26.

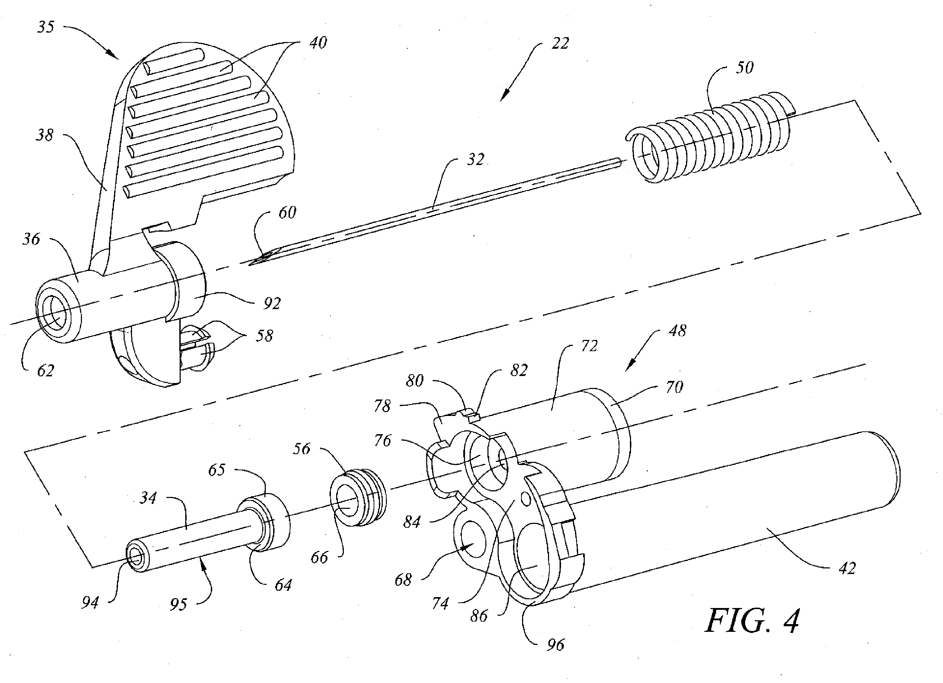

During or following the injection, a clinician administering the injection can grasp the textured surfaces of outwardly projecting stabilizer tab 38 of medical device 22 to stabilize medical device 22 and rotate syringe barrel 24, preferably in a clockwise direction (as viewed from the back of assembled combination 20), relative to stabilizer tab 38 of nose 35 (best seen in FIG. 4). Although medical device 22 of the invention can be made so as to permit rotation of syringe barrel 24 in either a clockwise or counter-clockwise direction relative to stabilizer tab 38 of nose 35 to achieve needle retraction, one or more mechanical barriers to counter-clockwise rotation of syringe barrel 24 are desirably provided to avoid accidentally unthreading luer lock connector 47, 49 of syringe barrel 20 from nose 35.

Satisfactory structural elements for use in combination 20 and in medical device 22 of the invention are further described and explained in relation to FIGS. 1-4. Medical device 22 as shown comprises nose 35, a retraction mechanism further comprising a needle holder 95 (FIG. 4) and a compressed retraction spring 50 seated inside nose 35, and hub assembly 48 comprising hub 72, frame member 74, retraction tube 42 further comprising retraction cavity 86, and an annular fluid seal such as fluid seal 56 that is desirably disposed between nose 35 and hub 72, preferably inside a recess 76 in the forwardly facing portion of hub 72. A plurality of radially projecting locking wedges 70, diametrically opposed as shown, or other similarly effective engagement elements are provided at the rear of hub 72 to facilitate locking engagement with locking luer connector 47, 49 of syringe barrel 24.

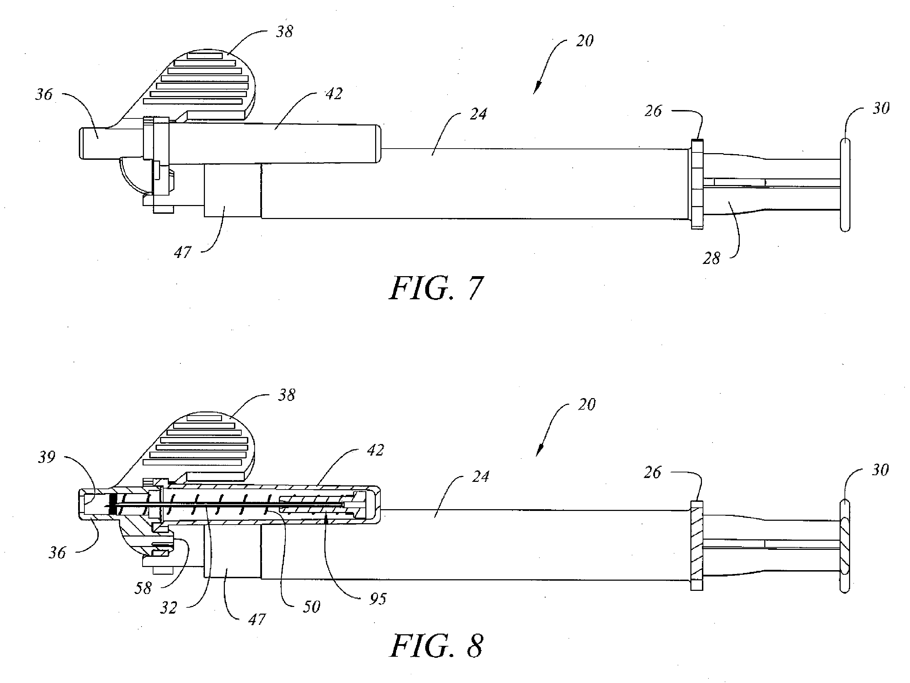

Referring to FIG. 4, needle holder 95 as shown comprises elongated cylindrical shaft portion 34, a larger diameter head 65, which is seated against an annular shoulder inside substantially cylindrical body 36 of nose when the retraction mechanism is seated inside nose 35 prior to attaching nose 35 to hub assembly 48 as further described below. Prior to seating needle holder 95 inside body 36 of nose 35, retraction spring 50 is desirably compressed (spring 50 is shown compressed in FIGS. 2-4 and relaxed in FIG. 8) between an annular nose 39 near the front of body 36 (best seen in FIG. 8) and annular shoulder 64 (FIG. 4) on the forwardly facing surface of head 65 of needle holder 95. The length of cylindrical shaft portion 34 of needle holder 95 is desirably such that the forwardly extending end of shaft portion 34 will project slightly beyond the front of body 36 as seen in FIG. 1.

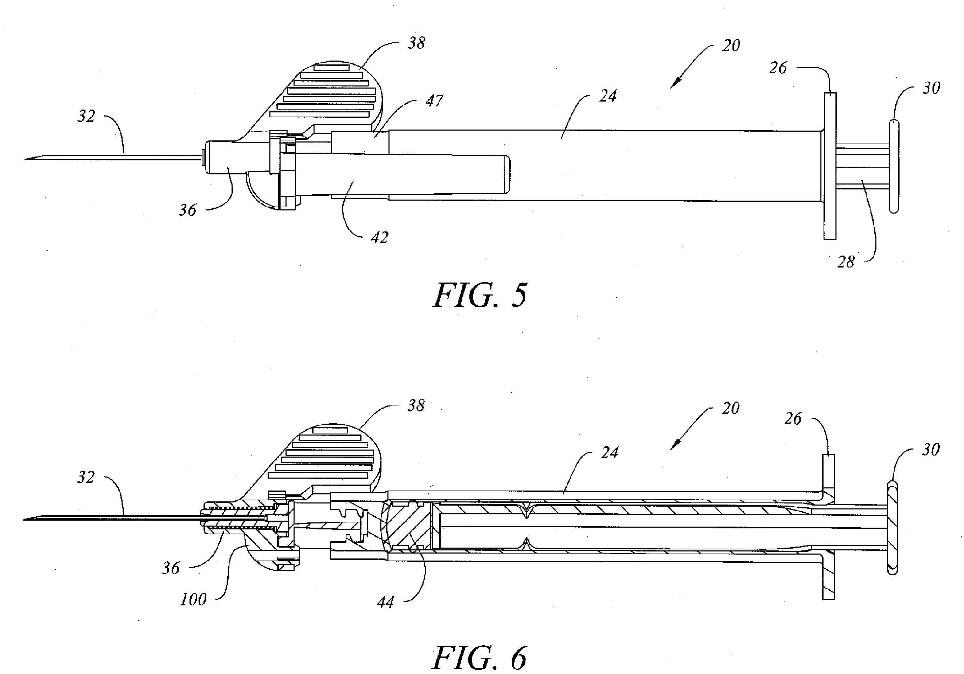

As shown, nose 35 further comprises substantially cylindrical body 36 having a rearwardly facing collar 92, and an outwardly projecting stabilizer tab 38 with textured gripping elements 40. Body 36 of nose 35 desirably further comprises an attachment tab 100 (FIG. 6) projecting radially outward below the longitudinal axis through interior cavity 62 that has a rearwardly projecting, split cylindrical boss 58 configured to snap into a cooperatively aligned orifice 68 in frame member 74 of hub assembly 48. Nose 35 is thereby rotatably attached to hub assembly 48 with the axis of rotation offset being offset from the longitudinal axes through hub 72 and retraction tube 42 so as to define an arc through which hub assembly 48 can be moved translationally from a first position characterized by coaxial alignment of hub 72 with body 36 to a second position characterized by coaxial alignment of retraction tube 42 with body 36 to initiate retraction following injection. Although the structural elements as disclosed are satisfactory for rotatably mounting nose 35 in relation to hub assembly 48, it will be appreciated by those of ordinary skill in the art upon reading this disclosure that other structural elements and configurations can be substituted for those particularly disclosed here to achieve the same functionality within the scope of the invention. Broadly stated, such functionality comprises rotating a syringe barrel following injection to move the barrel translationally relative to a nose portion comprising a retraction mechanism to initiate retraction of a needle from a patient into a cavity inside a retraction tube so that the tip of the needle no longer projects forwardly of the barrel.

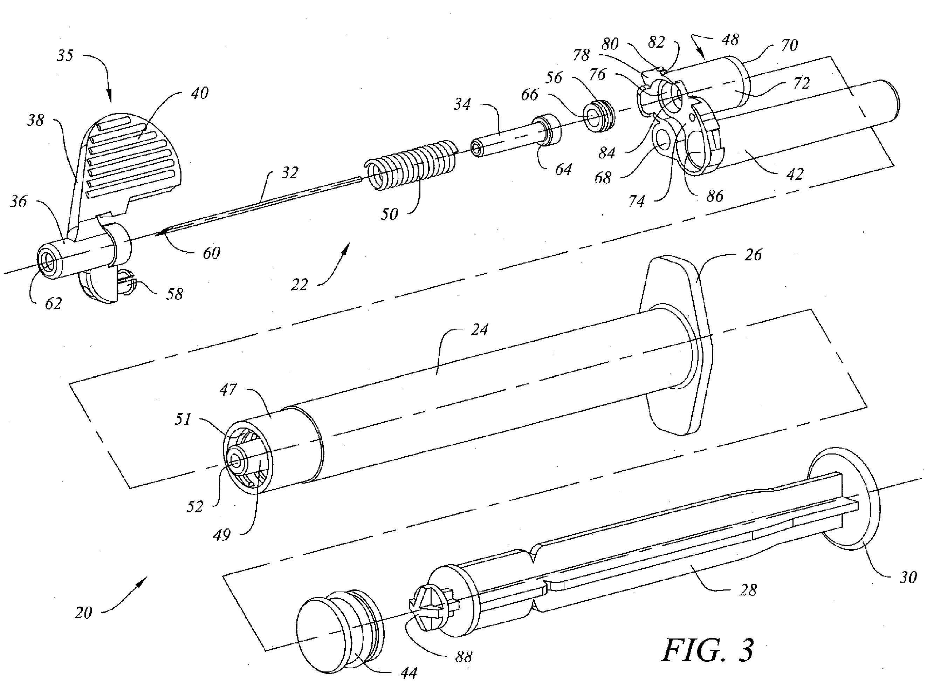

As shown in FIG. 3, a syringe suitable for use with medical device 22 of the invention can comprise barrel 24 with radially projecting flanges 26 disposed along the rear portion of barrel 24, an engagement structure such as luer lock connector 47, 49 attachable to hub assembly 48, plunger seal 44 attached to mounting boss 88 of plunger handle 28, and a thumb cap 30 to facilitate the application of a forwardly directed force to plunger handle 28 relative to barrel 24 during injection. As shown, tapered luer member 49 comprising a substantially cylindrical bore 51 cooperates with annular collar 47 having internal threads 51 disposed in spaced-apart relation to tapered luer member 49 to form luer lock connector 47, 49 that is engageable with locking wedges 70 of hub 72 to provide a fluid-tight seal between hub 72 and syringe barrel 24. When combination 20 is assembled as shown in FIG. 1, a continuous fluid flow path is formed from fluid chamber 46 (FIG. 2) through interior 84 of hub 72, interior 66 of fluid seal 56, interior 94 of needle holder 95, and needle 32 (FIG. 4).

Referring to FIGS. 5 and 6, the assembled combination 20 is again shown, but this time with plunger handle 28 advanced relative to syringe barrel 24 to the point it will be following an injection, when plunger seal 44 has forced the fluid out of fluid chamber 46 as previously shown and described in relation to FIGS. 1 and 2. This is the position in which plunger handle 28 will desirably remain relative to syringe barrel 24 during activation of the retraction mechanism and retraction of needle 32. By comparing the position of retraction tube 42 in FIG. 5 to that shown in FIG. 1, it is apparent that rotation of barrel 24 and retraction tube 42 relative to stabilization tab 38 of nose 35 has already been initiated.

Referring to FIGS. 7 and 8, the assembled combination 20 is again shown, and by comparing the position of retraction tube 42 in FIG. 7 to that shown in FIG. 5, it is apparent that rotation of barrel 24 and retraction tube 42 relative to stabilization tab 38 of nose 35 has continued to a point where retraction has occurred, and retraction spring 50 has propelled needle holder 95 in retraction tube 42 to a position where no portion of needle 32 is exposed forwardly of body 36.

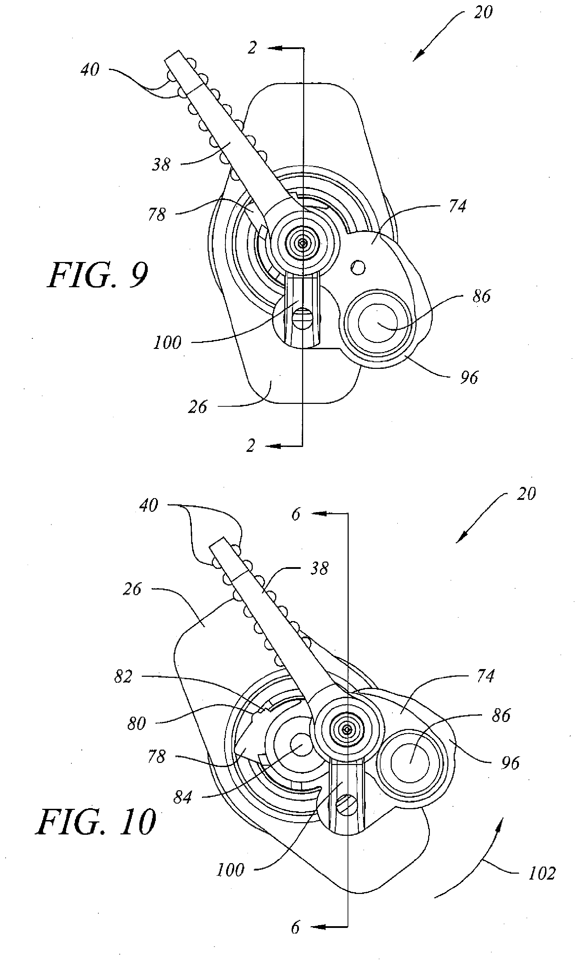

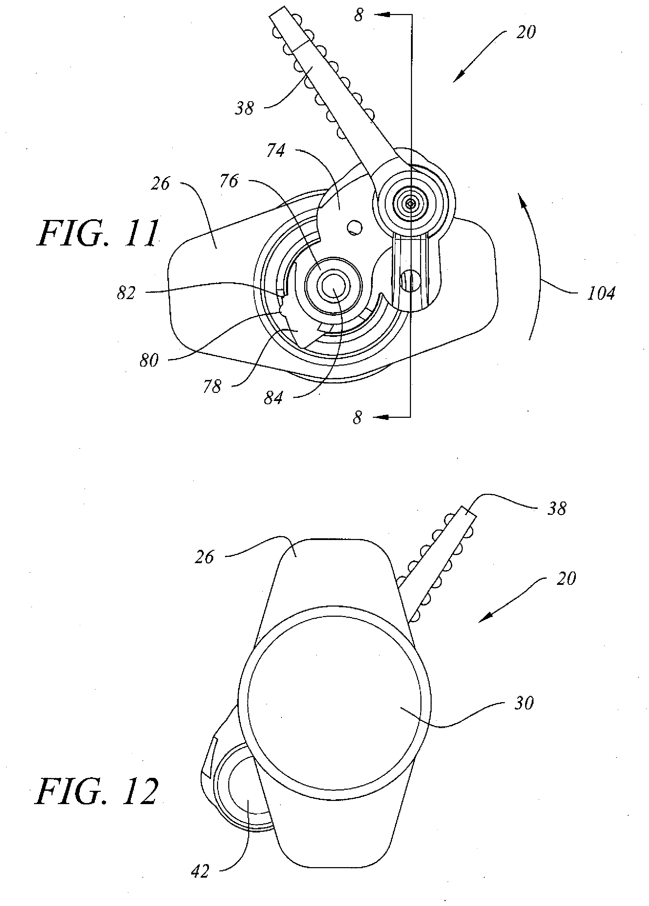

FIG. 9 depicts the relative positions of the components of combination 20 as in FIG. 1, but viewed from the front. FIG. 12 depicts the relative positions of the components of combination 20 as in FIG. 1, but viewed from the rear.

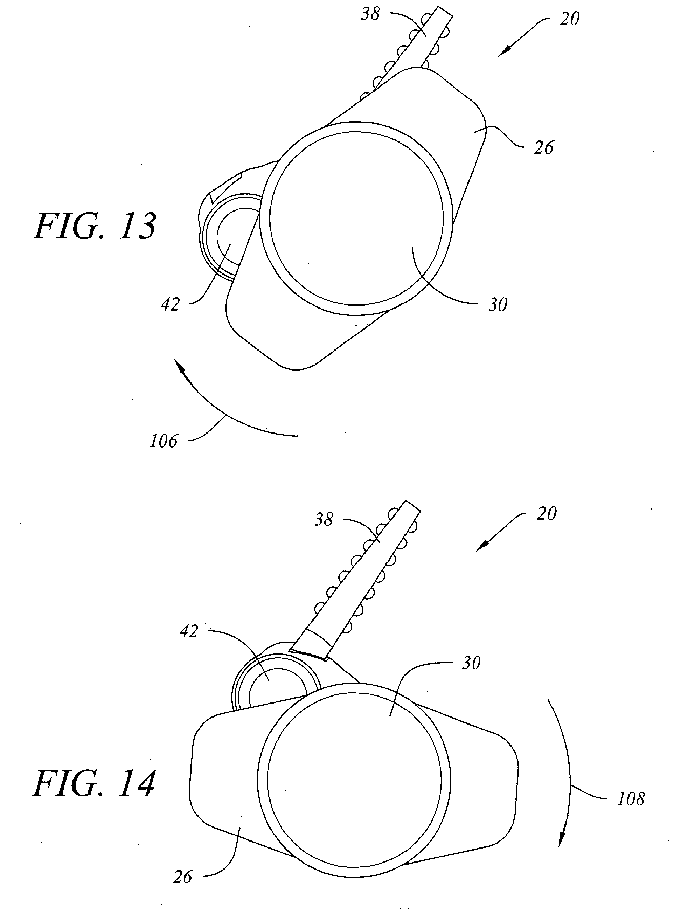

FIG. 10 depicts the relative positions of the components of combination 20 as in FIG. 5, but viewed from the front, with arrow 102 showing the direction of rotation of frame member 74 of hub assembly 48 relative to stabilization tab 38. FIG. 13 depicts the relative positions of the components of combination 20 as in FIG. 5, but viewed from the rear, with arrow 106 showing the direction of rotation of retraction tube 42 and syringe flanges 26 relative to stabilization tab 38.

FIG. 11 depicts the relative positions of the components of combination 20 as in FIG. 5, but viewed from the front, with arrow 104 showing the direction of rotation of frame member 74 of hub assembly 48 relative to stabilization tab 38. FIG. 14 depicts the relative positions of the components of combination 20 as in FIG. 5, but viewed from the rear, with arrow 108 showing the direction of rotation of retraction tube 42 and syringe flanges 26 relative to stabilization tab 38.

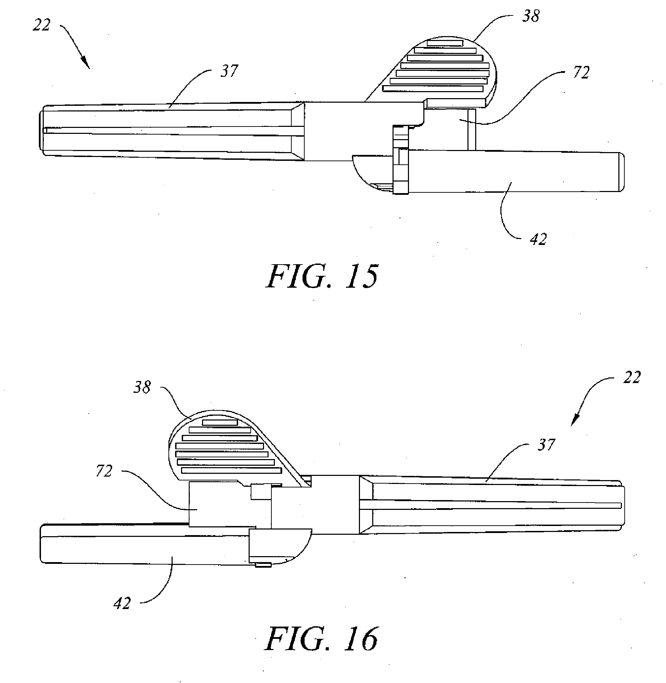

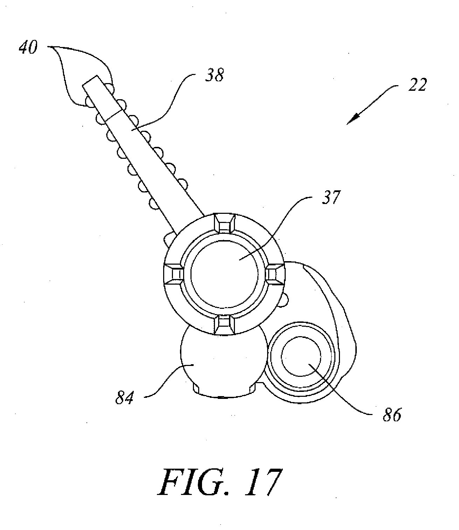

Nose 35, needle holder 95, hub assembly 48, syringe barrel 24 and plunger handle 28 are all desirably moldable from a suitable moldable polymeric material. Such materials and molding methods are believed to be well known to those of ordinary skill in the art. Similarly, it will be appreciated by those of skill in the art of syringe design and manufacture that a medical device such as medical device 22 disclosed here can be used with syringes that are either pre-filled or not, and that may comprise component portions made of glass or other suitable materials for particular applications. Similarly, it will be appreciated that fluid seal 56 and plunger seal 44 are desirably made of a rubbery or elastomeric polymeric material of the types commonly known for use in such medical applications. Similarly, it will be appreciated that materials used in the fabrication of this and other medical devices must be approved by the relevant regulatory authorities for use in such devices. Retraction spring 50 and needle 32 as disclosed are desirably made of stainless steel or any other similarly effective material. A needle cover 37 for medical device 22 is shown in FIGS. 15-17 for use with needle 32 while it is disposed in the forwardly projecting position prior to use, and it will be appreciated by those of skill in the art that such needle covers 37 are needed to protect needle 32 from contamination or damage during shipment and storage, and can be designed and fabricated using known technology.

Other alterations and modifications of the invention will likewise become apparent to those of ordinary skill in the art upon reading this specification in view of the accompanying drawings, and it is intended that the scope of the invention disclosed herein be limited only by the broadest interpretation of the appended claims to which the inventors are legally entitled.

## Claims

1. A medical device
selectively attachable to a syringe
having a barrel (24) with a front end and a back end, a plunger slidably disposed inside the barrel (24), a fluid chamber (46) disposed forwardly of the plunger, and a plunger handle (28) projecting rearwardly from an opening in the back end of the barrel (24),
the medical device comprising:
a hub assembly (48) comprising
a hub (72) which is disposable forwardly of the plunger,
a retraction tube (42) laterally spaced apart from the hub (72), and
a frame member (74) interconnecting the hub (72) and the retraction tube (42) in fixed, laterally spaced-apart relation to each other;
a nose (35) projecting forwardly from the hub (72);
a rearwardly biased needle retraction mechanism (95, 50) seated inside the nose (35);
a retractable needle (32) projecting forwardly of the nose (35);
a fluid flow path from the fluid chamber (46) through the hub (72), nose (35), needle retraction mechanism (95, 50) and needle (32);
**characterized in that**
the nose (35) is connected in rotatable relation to the frame member (74),
the medical device is configured **in that** the nose (35) and the hub assembly (48) are manually rotatable with respect to each other by a user through an arc from
a first position wherein hub (72) and the barrel (24) are coaxially aligned with the needle (32) and the needle retraction mechanism (95, 50) to
a second position wherein the retraction tube (42) is coaxially aligned with the needle (32) and
needle retraction mechanism (95, 50) to allow the needle retraction mechanism (95, 50) to force the needle (32) rearwardly into the retraction tube (42) so that the needle (32) no longer projects forwardly of the nose (35).

2. The medical device of claim 1, wherein the hub (72) comprises a rearwardly facing first attachment element (70) that is selectively engageable with a cooperating second attachment element (47, 49) disposed at the front of the barrel (24).

3. The medical device of claim 2, wherein the first and second attachment elements (47, 49, 70) comprise a luer lock connector, wherein the first attachment element (70) comprises a plurality of spaced-apart, radially projecting lugs, and/or wherein the second attachment element comprises at least one female thread disposed inside a collar projecting forwardly from the barrel.

4. The medical device of one of the preceding claims, wherein a fluid seal (56) is disposed between the nose (35) and the hub (72), preferably the fluid seal (56) is seated inside an annular recess in a front face of the hub (72) and/or is an elastomeric ring.

5. The medical device of one of the preceding claims, wherein the needle retraction mechanism (95, 50) comprises a needle holder (95) and a spring (50).

6. The medical device of claim 5, wherein the needle holder (95) is biased against a fluid seal (56) disposed between the nose (35) and the hub (72) whenever the barrel (24) and hub (72) assembly are disposed in the first position, and/or wherein the needle holder (95) is biased against the frame member (74) whenever the barrel and hub assembly are disposed between the first position and the second position, and/or
wherein the needle holder (95) is aligned with an opening in the front of the retraction tube (42) whenever the barrel and hub assembly are disposed in the second position, and/or wherein a portion of the needle holder (95) projects forwardly from the nose (35).

7. The medical device of claim 5, wherein the spring (50) is compressed between an annular surface in the front of the nose (35) and an annular shoulder (64) on the needle holder (95) whenever the barrel and hub assembly are disposed in the first position.

8. The medical device of one of the preceding claims, wherein the nose (35) further comprises a radially projecting stabilizer tab (38) that is grasped by a user to hold the nose (35) in a fixed position relative to a patient while rotating the barrel and hub assembly from the first position to the second position, preferably the radially projecting stabilizer tab (38) is textured to render it more graspable by a user.

9. The medical device of one of the preceding claims, wherein the frame member (74) comprises an orifice (68) disposed proximally to the hub (72) and to the retraction tube (42), and wherein the nose (35) comprises a rearwardly extending boss (58) that is inserted through the orifice (68) and snaps into rotatable engagement with the frame member (74).

10. The medical device of claim 8, wherein the nose (35) and the hub assembly (48) are cooperatively configured to allow rotation of the barrel and hub assembly in a clockwise direction relative to the nose as viewed from the back end of the barrel (24) when the stabilizer tab is grasped by the user while rotating the barrel and hub assembly from the first position to the second position, and/or
wherein the nose (35) and the hub assembly (48) are cooperatively configured to restrict rotation of the barrel and hub assembly in a counter-clockwise direction relative to the nose as (35) viewed from the back end of the barrel (24) when the stabilizer tab (38) is grasped by the user while rotating the barrel and hub assembly from the first position to the second position.

11. The medical device of one of the preceding claims, wherein the nose (35) and the hub assembly (48) are each made of moldable polymeric material and/or wherein the hub, retraction tube and frame member are integrally molded from a polymeric material.

12. The medical device of one of the preceding claims, configured for use with a syringe having a glass barrel and/or
configured for use with a syringe having a plastic barrel, and/or
configured for use with a prefilled syringe.

13. The medical device of one of the preceding claims, wherein needle retraction mechanism provides a retraction force upon activation that is sufficient to retract the needle directly from a patient into which the needle is inserted during an injection.

14. The medical device of one of the preceding claims in combination with a syringe attached to the hub assembly.

## Patentansprüche

1. Eine medizinische Vorrichtung die selektiv an einer Spritze mit einem Zylinder (24) mit einem vorderen Ende und einem hinteren Ende, einem Kolben, der verschiebbar innerhalb des Zylinders (24) angeordnet ist, einer Fluidkammer (46), die vor dem Kolben angeordnet ist, und einem Kolbengriff (28), der von einer Öffnung im hinteren Ende des Zylinders (24) nach hinten vorsteht, befestigbar ist,
wobei die medizinische Vorrichtung umfasst:
eine Nabenanordnung (48) mit
eine Nabe (72), die vor dem Kolben angeordnet werden kann
ein Rückzugsrohr (42), das seitlich von der Nabe (72) beabstandet ist, und
ein Rahmenelement (74), das die Nabe (72) und das Rückzugsrohr (42) in fester, seitlich beabstandeter Beziehung zueinander miteinander verbindet;
eine Nase (35), die von der Nabe (72) nach vorne vorsteht;
einen nach hinten vorgespannten Nadelrückzugsmechanismus (95, 50), der innerhalb der Nase (35) sitzt;
eine einziehbare Nadel (32), die von der Nase (35) nach vorne vorsteht;
einen Fluidströmungsweg von der Fluidkammer (46) durch die Nabe (72), die Nase (35), den Nadelrückzugsmechanismus (95, 50) und die Nadel (32);
**dadurch gekennzeichnet, dass**
die Nase (35) drehbar mit dem Rahmenelement (74) verbunden ist,
die medizinische Vorrichtung so konfiguriert ist, dass die Nase (35) und die Nabenanordnung (48) durch einen Benutzer manuell in Bezug aufeinander über einen Bogen von einer ersten Position, in der die Nabe (72) und der Zylinder (24) koaxial mit der Nadel (32) und dem Nadelrückzugsmechanismus (95, 50) ausgerichtet sind, zu einer zweiten Position, in der das Rückzugsrohr (42) koaxial mit der Nadel (32) und dem Nadelrückzugsmechanismus (95, 50) ausgerichtet ist, damit der Nadelrückzugsmechanismus (95, 50) die Nadel (32) nach hinten in das Rückzugsrohr (42) zwingen kann, so dass die Nadel (32) nicht mehr über die Nase (35) hinausragt, rotiert werden kann.

2. Medizinische Vorrichtung nach Anspruch 1, wobei die Nabe (72) ein nach hinten gerichtetes erstes Befestigungselement (70) aufweist, das selektiv mit einem zusammenwirkenden zweiten Befestigungselement (47, 49) in Eingriff gebracht werden kann, das an der Vorderseite des Zylinders (24) angeordnet ist.

3. Medizinische Vorrichtung nach Anspruch 2, wobei das erste und das zweite Befestigungselement (47, 49, 70) einen Luer-Lock-Verbinder umfassen, wobei das erste Befestigungselement (70) eine Vielzahl von beabstandeten, radial vorstehenden Nasen umfasst, und/oder wobei das zweite Befestigungselement mindestens ein Innengewinde umfasst, das innerhalb eines Kragens angeordnet ist, der von dem Zylinder nach vorne vorsteht.

4. Medizinische Vorrichtung nach einem der vorhergehenden Ansprüche, bei der eine Flüssigkeitsdichtung (56) zwischen der Nase (35) und der Nabe (72) angeordnet ist, wobei die Flüssigkeitsdichtung (56) vorzugsweise in einer ringförmigen Aussparung in einer Vorderfläche der Nabe (72) sitzt und/oder ein Elastomerring ist.

5. Medizinische Vorrichtung nach einem der vorhergehenden Ansprüche, wobei der Nadelrückzugsmechanismus (95, 50) einen Nadelhalter (95) und eine Feder (50) umfasst.

6. Medizinische Vorrichtung nach Anspruch 5, wobei der Nadelhalter (95) gegen eine Fluiddichtung (56) vorgespannt ist, die zwischen der Nase (35) und der Nabe (72) angeordnet ist, wenn die Anordnung aus Zylinder (24) und Nabe (72) in der ersten Position angeordnet ist, und/oder wobei der Nadelhalter (95) gegen das Rahmenelement (74) vorgespannt ist, wenn die Zylinder- und Nabenanordnung zwischen der ersten Position und der zweiten Position angeordnet ist, und/oder
wobei der Nadelhalter (95) mit einer Öffnung in der Vorderseite des Rückzugsrohrs (42) ausgerichtet ist, wenn die Zylinder- und Nabenanordnung in der zweiten Position angeordnet ist, und/oder
wobei ein Teil des Nadelhalters (95) von der Nase (35) nach vorne vorsteht.

7. Medizinische Vorrichtung nach Anspruch 5, wobei die Feder (50) zwischen einer ringförmigen Fläche an der Vorderseite der Nase (35) und einer ringförmigen Schulter (64) am Nadelhalter (95) zusammengedrückt wird, wenn sich die die Zylinder- und Nabenanordnung in der ersten Position befindet.

8. Medizinische Vorrichtung nach einem der vorhergehenden Ansprüche, wobei die Nase (35) ferner eine radial vorstehende Stabilisierungslasche (38) aufweist, die von einem Benutzer ergriffen wird, um die Nase (35) in einer festen Position relativ zu einem Patienten zu halten, während die Zylinder- und Nabenanordnung von der ersten Position in die zweite Position gedreht wird, wobei die radial vorstehende Stabilisierungslasche (38) vorzugsweise strukturiert ist, um sie für einen Benutzer besser greifbar zu machen.

9. Medizinische Vorrichtung nach einem der vorhergehenden Ansprüche, wobei das Rahmenelement (74) eine Öffnung (68) aufweist, die proximal zur Nabe (72) und zum Rückzugsrohr (42) angeordnet ist, und wobei die Nase (35) einen sich nach hinten erstreckenden Vorsprung (58) aufweist, der durch die Öffnung (68) eingeführt wird und in drehbaren Eingriff mit dem Rahmenelement (74) einrastet.

10. Medizinische Vorrichtung nach Anspruch 8, wobei die Nase (35) und die Nabenanordnung (48) zusammenwirkend so konfiguriert sind, dass sie eine Drehung der Zylinder- und Nabenanordnung im Uhrzeigersinn relativ zur Nase, vom hinteren Ende des Zylinders (24) aus gesehen, ermöglichen, wenn die Stabilisierungslasche vom Benutzer ergriffen wird, während die Zylinder-und Nabenanordnung von der ersten Position in die zweite Position gedreht wird, und/oder wobei die Nase (35) und die Nabenanordnung (48) zusammenwirkend so konfiguriert sind, dass sie die Drehung der Zylinder- und Nabenanordnung gegen den Uhrzeigersinn relativ zur Nase (35), vom hinteren Ende des Laufs (24) aus gesehen, einschränken, wenn die Stabilisierungslasche (38) vom Benutzer ergriffen wird, während die Zylinder- und Nabenanordnung von der ersten Position in die zweite Position gedreht wird.

11. Medizinische Vorrichtung nach einem der vorhergehenden Ansprüche, wobei die Nase (35) und die Nabenanordnung (48) jeweils aus einem formbaren Polymermaterial hergestellt sind und/oder wobei die Nabe, das Rückzugsrohr und das Rahmenteil einstückig aus einem Polymermaterial geformt sind.

12. Medizinische Vorrichtung nach einem der vorhergehenden Ansprüche, konfiguriert zur Verwendung mit einer Spritze mit einem Glaszylinder und/oder
zur Verwendung mit einer Spritze mit einem Kunststoffzylinder, und/oder
konfiguriert zur Verwendung mit einer vorgefüllten Spritze.

13. Medizinische Vorrichtung nach einem der vorhergehenden Ansprüche, wobei der Nadelrückzugsmechanismus bei Aktivierung eine Rückzugskraft bereitstellt, die ausreicht, um die Nadel direkt von einem Patienten, in den die Nadel während einer Injektion eingeführt wird, zurückzuziehen.

14. Medizinische Vorrichtung nach einem der vorhergehenden Ansprüche in Kombination mit einer an der Nabenanordnung befestigten Spritze.

## Revendications

1. Dispositif médical
pouvant être fixé de manière sélective à une seringue ayant un cylindre (24) avec une extrémité avant et une extrémité arrière, un piston disposé de manière à pouvoir coulisser à l'intérieur du cylindre (24), une chambre de fluide (46) disposée vers l'avant du piston, et une poignée de piston (28) se projetant vers l'arrière depuis une ouverture dans l'extrémité arrière du cylindre (24),
le dispositif médical comprenant :
un ensemble embase (48) comprenant
une embase (72) qui peut être disposée vers l'avant du piston,
un tube de rétractation (42) espacé latéralement à l'écart de l'embase (72), et
un élément formant cadre (74) interreliant l'embase (72) et le tube de rétractation (42) dans une relation fixe, latéralement espacés à l'écart l'un de l'autre ;
un nez (35) se projetant vers l'avant de l'embase (72) ;
un mécanisme de rétractation d'aiguille (95, 50) sollicité vers l'arrière logé à l'intérieur du nez (35) ;
une aiguille (32) rétractable se projetant vers l'avant du nez (35) ;
un trajet d'écoulement de fluide depuis la chambre de fluide (46) à travers l'embase (72), le nez (35), le mécanisme de rétractation d'aiguille (95, 50) et l'aiguille (32) ;
**caractérisé en ce que**
le nez (35) est relié dans une relation permettant de tourner à l'élément formant cadre (74),
le dispositif médical étant conçu **en ce que** le nez (35) et l'ensemble embase (48) peuvent être manuellement tournés l'un par rapport à l'autre par un utilisateur à travers un arc depuis une première position dans laquelle l'embase (72) et le cylindre (24) sont alignés de manière coaxiale avec l'aiguille (32) et le mécanisme de rétractation d'aiguille (95, 50) jusqu'à une seconde position dans laquelle le tube de rétractation (42) est aligné de manière coaxiale avec l'aiguille (32) et le mécanisme de rétractation d'aiguille (95, 50) pour permettre au mécanisme de rétractation d'aiguille (95, 50) de forcer l'aiguille (32) vers l'arrière dans le tube de rétractation (42) de sorte que l'aiguille (32) ne se projette plus vers l'avant du nez (35).

2. Dispositif médical selon la revendication 1, l'embase (72) comprenant un premier élément de fixation (70) faisant face vers l'arrière qui peut être sélectivement engagé avec un second élément de fixation (47, 49) en coopération disposé à l'avant du cylindre (24).

3. Dispositif médical selon la revendication 2, le premier et le second élément de fixation (47, 49, 70) comprenant un raccord à verrouillage Luer, le premier élément de fixation (70) comprenant une pluralité de crans espacés à l'écart, se projetant radialement, et/ou le second élément de fixation comprenant au moins un filetage femelle disposé à l'intérieur d'un collier se projetant vers l'avant depuis le cylindre.

4. Dispositif médical selon l'une des revendications précédentes, un joint de fluide (56) étant disposé entre le nez (35) et l'embase (72), préférablement le joint de fluide (56) étant logé à l'intérieur d'un renfoncement annulaire dans une face avant de l'embase (72) et/ou étant un anneau élastomère.

5. Dispositif médical selon l'une des revendications précédentes, le mécanisme de rétractation d'aiguille (95, 50) comprenant un support d'aiguille (95) et un ressort (50).

6. Dispositif médical selon la revendication 5, le support d'aiguille (95) étant sollicité contre un joint de fluide (56) disposé entre le nez (35) et l'embase (72) à chaque fois que l'ensemble cylindre (24) et embase (72) sont disposés dans la première position, et/ou le support d'aiguille (95) étant sollicité contre l'élément formant cadre (74) à chaque fois que l'ensemble cylindre et embase sont disposés entre la première position et la seconde position, et/ou
le support d'aiguille (95) étant aligné avec une ouverture dans l'avant du tube de rétractation (42) à chaque fois que l'ensemble cylindre et embase sont disposés dans la seconde position, et/ou
une portion du support d'aiguille (95) se projetant vers l'avant depuis le nez (35).

7. Dispositif médical selon la revendication 5, le ressort (50) étant comprimé entre une surface annulaire dans l'avant du nez (35) et un épaulement annulaire (64) sur le support d'aiguille (95) à chaque fois que l'ensemble cylindre et embase sont disposés dans la première position.

8. Dispositif médical selon l'une des revendications précédentes, le nez (35) comprenant en outre un onglet de stabilisation (38) se projetant radialement qui est saisi par un utilisateur pour tenir le nez (35) dans une position fixe par rapport à un patient tout en tournant l'ensemble cylindre et embase depuis la première position vers la seconde position, préférablement l'onglet de stabilisation (38) qui se projette radialement étant texturé pour le rendre plus facile à saisir par un utilisateur.

9. Dispositif médical selon l'une des revendications précédentes, l'élément formant cadre (74) comprenant un orifice (68) disposé de manière proximale par rapport à l'embase (72) et au tube de rétractation (42), et le nez (35) comprenant une bosse (58) s'étendant vers l'arrière qui est insérée à travers l'orifice (68) et en ajustement par pression en engagement pouvant tourner avec l'élément formant cadre (74).

10. Dispositif médical selon la revendication 8, le nez (35) et l'ensemble embase (48) étant conçus de manière à coopérer pour permettre une rotation de l'ensemble cylindre et embase dans un sens des aiguilles d'une montre par rapport au nez tel que visualisé depuis l'extrémité arrière du cylindre (24) lorsque l'onglet de stabilisation est saisi par l'utilisateur tout en faisant tourner l'ensemble cylindre et embase depuis la première position vers la seconde position, et/ou
le nez (35) et l'ensemble embase (48) étant conçus de manière à coopérer pour restreindre une rotation de l'ensemble cylindre et embase dans un sens contraire aux aiguilles d'une montre par rapport au nez (35) tel que visualisé depuis l'extrémité arrière du cylindre (24) lorsque l'onglet de stabilisation (38) est saisi par l'utilisateur tout en tournant l'ensemble cylindre et embase depuis la première position vers la seconde position.

11. Dispositif médical selon l'une des revendications précédentes, le nez (35) et l'ensemble embase (48) étant chacun constitués d'un matériau polymère pouvant être moulé et/ou l'embase, le tube de rétractation et l'élément formant cadre étant intégralement moulés à partir d'un matériau polymère.

12. Dispositif médical selon l'une des revendications précédentes, conçu pour l'utilisation avec une seringue ayant un cylindre en verre et/ou
conçu pour l'utilisation avec une seringue ayant un cylindre en plastique, et/ou
conçu pour l'utilisation avec une seringue préremplie.

13. Dispositif médical selon l'une des revendications précédentes, le mécanisme de rétractation d'aiguille fournissant une force de rétractation lors d'une activation qui est suffisante pour rétracter l'aiguille directement d'un patient dans lequel l'aiguille est insérée durant une injection.

14. Dispositif médical selon l'une des revendications précédentes en combinaison avec une seringue fixée à l'ensemble embase.
